# EUROPEAN PATENT SPECIFICATION

(11) **EP 1 402 819 B1**
(45) Date of publication and mention of the grant of the patent: **15.02.2006**
(21) Application number: 03388061.8
(22) Date of filing: 17.09.2003
(51) Int. Cl.: A61B 8/12

(54) **Catheter for insertion into the human body**
Katheter zur Einführung in den menschlichen Körper
Cathéter pour insertion dans le corps humain

(30) Priority: 25.09.2002 DK 200201419
(43) Date of publication of application: 31.03.2004
(73) Proprietor: B-K Medical ApS, 2730 Herlev (DK)
(72) Inventor: Nygaard, Per Ehrenreich, 2860 Soeborg (DK); Olsen, Tommy Björn, 3600 Frederikssund (DK); Lange, Niels Henrik, 3050 Humlabaek (DK)
(74) Representative: Siiger, Joergen

(56) References cited:
- US-A- 5 025 778
- US-A- 6 004 273
- US-A- 6 083 169
- US-A1- 2001 047 165

## Description

The invention relates to a catheter for insertion into the human body and which includes one or more optionally scanning ultrasonic transducers as well as a surgical instrument to be operated from the outside.

Many catheters are known for insertion into the human body in order to extract tissue samples. Many of these known catheters are encumbered with the draw-back that they are difficult to clean and disinfect upon use.

According to Danish Patent Application No. 12/98 attempts have been made at solving this problem by forming the needle guide separately relative to the remaining part of the catheter which has been coated with a sterile sheath. Then the needle guide is secured to the remaining part of the catheter on the outer side of the sheath in such a manner that the needle need not penetrate said sheath during a sampling. As a result it is not necessary to disinfect the catheter upon use. However, great interest attaches in avoiding such a sheath.

US A 5,025,778 discloses a catheter comprising parts of a circular cross section.

The object of the invention is therefore to provide a catheter of the above type which is easier to disinfect than hitherto known and which therefore does not need such a sheath. The object of the invention is solved by a catheter according to claim 1.

As a result, the catheter includes only very few parts which in the separated state are easy to clean and disinfect.

Moreover, the surgical instrument may according to the invention be formed by a flexible needle for the introduction of a substance or for the extraction of tissue samples.

In addition, the longitudinal groove in the surface of one of the partially circular parts may be longer than the surrounding outer tube and be shaped so that the flexible needle extends immediately behind said outer surrounding tube.

The invention is explained in detail below with reference to the drawings, in which
Figure 1 shows a catheter according to the invention which includes two parts of a semicircular cross section which are kept together by means of an outer tube,
Figure 2 is a longitudinal sectional view from the outside of the two parts of a semicircular cross section,
Figure 3 shows the outer tube,
Figure 4 shows a catheter housing a surgical instrument in form of a needle for the introduction of a substance or for the extraction of a tissue sample and
Figure 5 shows a sigmoidoscope.

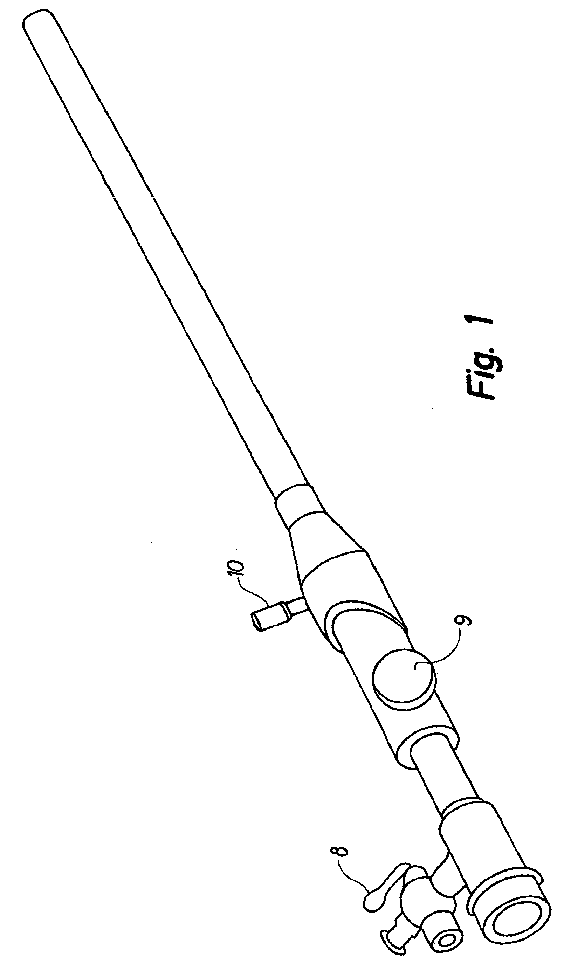

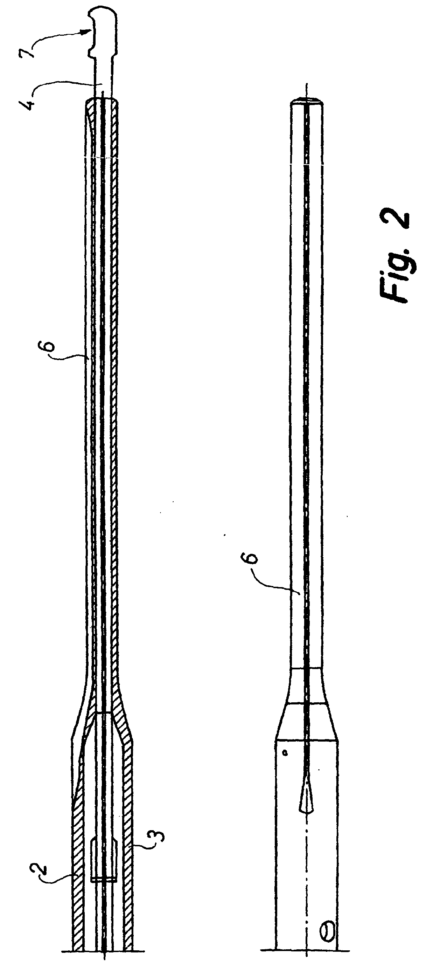

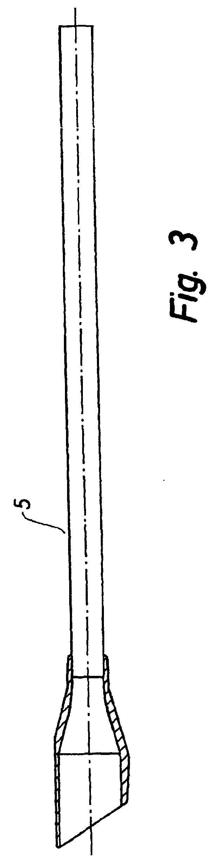

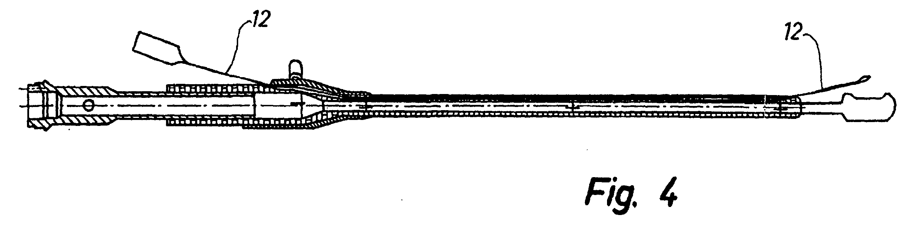

Figures 1-4 show a catheter according to the invention for insertion into the human body. The catheter includes one or more parts 2, 3 of a partially circular cross section, said parts preferably being two substantially semicircular parts. As an alternative that is not part of the invention, the one or more parts 2, 3 may be of a substantially completely circular cross section. A rod 4 is inserted between these parts 2, 3, said rod being provided at the projecting end with an ultrasonic transducer 7. The rod 4 can be rotated relative to the two semicircular parts 2, 3. The two semicircular parts 2, 3 are kept together by means of an outer tube 5 passed over said two semicircular parts 2, 3. The abutting surfaces of the two semicircular parts are formed such that they lock relative to one another. A longitudinal groove 6 is provided in the surface of at least one semicircular part 2, said groove allowing the insertion of a surgical instrument, such as a flexible needle or a cannula 12 for a sampling or for the introduction of a substance. The groove 6 is longer than the outer tube 5 and shaped such that the cannula 12 is positioned so as to extend immediately behind the outer tube 5, said groove 6 ending in the surface of the semicircular part 2 immediately behind the outer tube 5. During the extraction of a tissue or liquid sample from the human body the cannula 12 can then be observed by means of the ultrasonic transducer 7 in form of the location where said cannula 12 passes the transverse plane being scanned by said transducer 7. The observation can for instance be displayed on a screen.

The catheter is furthermore equipped with a valve 8 for the introduction of a liquid, preferably brine. The brine exits at the opposite end where the needle 12 extends out of the catheter in such a manner that an acoustic impedance matching always applies to the tissue to be examined.

In addition, means 9 can be provided for fixing the rod 4 relative to the two semicircular parts 2, 3, as well as a screw 10 can be provided for securing the outer tube 5 relative to the semicircular parts 2, 3.

The catheter can for instance be used for rectal purposes, such as in connection with rectoscopy.

The catheter is made of stainless steel and presents an outside diameter of approximately 6 to 24 mm.

The catheter can optionally be made of plastics or a combination of stainless steel and plastics.

In addition, the catheter is very user-friendly as well as easy to disassemble for disinfecting and sterilising purposes.

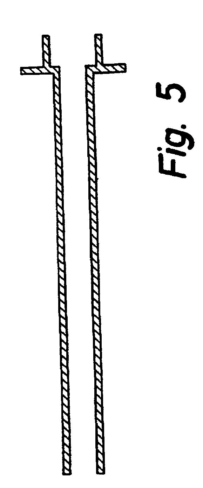

Another embodiment, a so-called sigmoidoscope or a rectoscope, includes a plastic or steel tube, cf. Figure 5. The plastic tube is then used as light conductor which renders it possible to introduce a telescope into the plastic tube which then exposes the surrounding tissue. It is possible to insert a cannula for a sampling in the rim of the above plastic tube. The catheter according to the invention can optionally be inserted in the above plastic tube upon detection of the various internal organs by means of the telescope.

## Claims

1. A catheter for insertion into the human body and which includes one or more optionally scanning ultrasonic transducers as well as a surgical instrument to be operated from the outside, **characterised by** comprising at least two parts (2, 3) of a partially circular cross section, a rotatable rod (4) being inserted between said by at least two parts (2, 3) of a partially circular cross section, and at the end being provided with an ultrasonic transducer (7), said partially circular parts (2, 3) being surrounded by a removable outer tube (5) passed over said partially circular parts (2, 3), the surface of one of said partially circular parts (2) being provided with a longitudinal groove (6) for insertion of the surgical instrument.

2. A catheter according to claim 1, **characterised in that** the surgical instrument is formed by a flexible needle (12) for the introduction of a substance or extraction of tissue samples.

3. A catheter according to claim 1, **characterised in that** a longitudinal groove (6) in the surface of one of the partially circular parts (2) is longer than the surrounding outer tube (5) and is shaped such that the flexible needle (12) extends immediately behind said outer surrounding tube (5).

4. A catheter according to claim 3, **characterised in that** the groove (6) in the surface of one of the partially circular parts (2) ends in the surface of the partially circular part (2) immediately behind said surrounding tube (5).

## Patentansprüche

1. Katheter zur Einführung in den menschlichen Körper, der einen oder mehrere Ultraschallwandler mit optionaler Abtastung und ein chirurgisches Instrument aufweist, die von außen bedient werden können, **gekennzeichnet durch** mindestens zwei Teile (2, 3) mit einem teilweise kreisförmigen Querschnitt, einem drehbaren Stab (4), der zwischen die mindestens zwei Teile (2, 3) mit einem teilweise kreisförmigen Querschnitt eingeführt ist und am Ende mit einem Ultraschallwandler (7) versehen ist, wobei die teilweise kreisförmigen Teile (2, 3) von einer abnehmbaren äußeren Röhre (5) umgeben sind, die auf die teilweise kreisförmigen Teile (2, 3) geschoben sind, wobei die Oberfläche des einen der kreisförmigen Teile (2) mit einer Längsnut (6) zur Einführung des chirurgischen Instruments versehen ist.

2. Katheter nach Anspruch 1, **dadurch gekennzeichnet, daß** das chirurgische Instrument durch eine flexible Nadel (12) zum Einführen einer Substanz oder zur Entnahme von Gewebeproben gebildet wird.

3. Katheter nach Anspruch 1, **dadurch gekennzeichnet, daß** die Längsnut (6) in der Oberfläche eines der teilweise kreisförmigen Teile (2) länger ist als die umgebende äußere Röhre (5) und so geformt ist, daß sich die flexible Nadel (12) unmittelbar hinter der äußeren umgebenden Röhre (5) erstreckt.

4. Katheter nach Anspruch 3, **dadurch gekennzeichnet, daß** die Nut (6) in der Oberfläche eines der teilweise kreisförmigen Teile (2) in der Oberfläche des teilweise kreisförmigen Teils (2) unmittelbar hinter der umgebenden Röhre (5) endet.

## Revendications

1. Cathéter destiné à être inséré dans le corps humain et qui comprend un ou plusieurs capteurs ultrasonores facultativement de balayage ainsi qu'un instrument chirurgical destiné à être actionné depuis l'extérieur, **caractérisé en ce qu'**il comprend au moins deux parties (2, 3) de section transversale partiellement circulaire, une tige rotative (4) qui est insérée entre lesdites au moins deux parties (2, 3) de section transversale partiellement circulaire et au niveau de l'extrémité est prévu avec un capteur ultrasonore (7), lesdites parties (2, 3) partiellement circulaires étant entourées par un tube externe (5) amovible qui passe sur lesdites parties (2, 3) partiellement circulaires, la surface de l'une desdites parties (2) partiellement circulaires étant prévue avec une rainure longitudinale (6) pour l'insertion de l'instrument chirurgical.

2. Cathéter selon la revendication 1, **caractérisé en ce que** l'instrument chirurgical est formé avec une aiguille (12) flexible pour l'introduction d'une substance ou l'extraction d'échantillons de tissu.

3. Cathéter selon la revendication 1, **caractérisé en ce qu'**une rainure longitudinale (6) dans la surface de l'une des parties (2) partiellement circulaires est plus longue que le tube externe (5) périphérique et est formée de sorte que l'aiguille (12) flexible s'étend immédiatement derrière ledit tube externe (5) périphérique.

4. Cathéter selon la revendication 3, **caractérisé en ce que** la rainure (6) dans la surface de l'une des parties (2) partiellement circulaires se termine dans la surface de la partie (2) partiellement circulaire immédiatement derrière ledit tube (5) périphérique.
